(19) Europäisches Patentamt
European Patent Office
Office européen des brevets

(11) **EP 4 360 102 B1**

(12) **EUROPEAN PATENT SPECIFICATION**

(45) Date of publication and mention
of the grant of the patent:
**24.12.2025   Bulletin 2025/52**

(21) Application number: **22743597.1**

(22) Date of filing: **20.06.2022**

(51) International Patent Classification (IPC):
***G16H 30/40*** (2018.01)        ***G16H 50/30*** (2018.01)

(52) Cooperative Patent Classification (CPC):
**G16H 30/40; G16H 50/30**

(86) International application number:
**PCT/IL2022/050658**

(87) International publication number:
**WO 2022/269603 (29.12.2022 Gazette 2022/52)**

(54) **SYSTEMS AND METHODS FOR ASSESSING GASTROINTESTINAL CLEANSING**

SYSTEME UND VERFAHREN ZUR BEURTEILUNG DER MAGEN-DARM-REINIGUNG

SYSTÈMES ET PROCÉDÉS D'ÉVALUATION DE NETTOYAGE GASTRO-INTESTINAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **24.06.2021   US 202163214338 P**

(43) Date of publication of application:
**01.05.2024   Bulletin 2024/18**

(73) Proprietor: **Given Imaging Ltd.**
**20692 Yoqneam (IL)**

(72) Inventors:
• **DEKEL, Eyal**
**20692 Yoqneam (IL)**

• **NIV, Eva**
**20692 Yoqneam (IL)**
• **EILOT, Dov**
**20692 Yoqneam (IL)**
• **PELEG, Dori**
**20692 Yoqneam (IL)**

(74) Representative: **HGF**
**HGF Limited**
**4th Floor, 1 City Square**
**Leeds LS1 2AL (GB)**

(56) References cited:
**WO-A1-2020/079696        CN-A- 110 916 606**
**US-B1- 8 792 691        US-B1- 8 861 783**

## Description

FIELD

The disclosure relates to image analysis of a stream of in-vivo images of a gastrointestinal tract (GIT) and, more particularly, to systems and methods for assessing degree of cleansing of a GIT or of portions of a GIT.

BACKGROUND

Capsule endoscopy (CE) allows examining the entire GIT endoscopically. There are capsule endoscopy systems and methods that are aimed at examining a specific portion of the GIT, such as the small bowel (SB) or the colon. CE is a non-invasive procedure which does not require the patient to be admitted to a hospital, and the patient can continue most daily activities while the capsule is in his body.

On a typical CE procedure, the patient is referred to a procedure by a physician. The patient then arrives at a medical facility (e.g., a clinic or a hospital), to perform the procedure. The capsule, which is about the size of a multi-vitamin, is swallowed by the patient under the supervision of a health professional (e.g., a nurse or a physician) at the medical facility and the patient is provided with a wearable device, e.g., a sensor belt and a recorder placed in a pouch and strap to be placed around the patient's shoulder. The wearable device typically includes a storage device. The patient may be given guidance and/or instructions and then released to his daily activities.

The capsule captures images as it travels naturally through the GIT. Images and additional data (e.g., metadata) are then transmitted to the recorder that is worn by the patient. The capsule is typically disposable and passes naturally with a bowel movement. The procedure data (e.g., the captured images or a portion of them and additional metadata) is stored on the storage device of the wearable device.

The wearable device is typically returned by the patient to the medical facility with the procedure data stored thereon. The procedure data is then downloaded to a computing device typically located at the medical facility, which has an engine software stored thereon. The received procedure data is then processed by the engine to a compiled study (or "study"). Typically, a study includes thousands of images (around 6,000). Typically, the number of images to be processed is of the order of tens of thousands and about 90,000 on average.

A reader (which may be the procedure supervising physician, a dedicated physician, or the referring physician) may access the study via a reader application. The reader then reviews the study, evaluates the procedure, and provides his input via the reader application. Since the reader needs to review thousands of images, the reading time of a study may usually take between half an hour to an hour on average and the reading task may be tiresome. A report is then generated by the reader application based on the compiled study and the reader's input. On average, it would take an hour to generate a report. The report may include, for example, images of interest, e.g., images which are identified as including pathologies, selected by the reader; evaluation or diagnosis of the patient's medical condition based on the procedure's data (i.e., the study) and/or recommendations for follow up and/or treatment provided by the reader. The report may be then forwarded to the referring physician. The referring physician may decide on a required follow up or treatment based on the report. In this context, patent document WO 2020/079696 A1 discloses a method for analysis of images taken by a capsule endoscope, by determining a cleanliness score for each image and displaying the score graphically along a representation of the gastrointestinal tract. Document WO 2020/079696 A1 does not disclose determining an average cleanliness score for a segment of the GI tract based on advancement information indicative of physical advancement of the capsule.

SUMMARY OF THE INVENTION

The invention is set out in the appended claims.

SUMMARY OF RELATED DISCLOSURE

To the extent consistent, any or all of the aspects detailed herein may be used in conjunction with any or all of the other aspects detailed herein. Aspects of the present disclosure relate to use of image processing to assess a degree of cleansing of a gastrointestinal tract (GIT) or of portions of a GIT. As persons skilled in the art will understand, cleansing refers to preparing a gastrointestinal tract to be optically imaged by clearing various matter and/or obstructions (e.g., bubbles) from the GIT or from portions of the GIT. Indicating the degree of cleansing of a GIT or of portions of a GIT may help physicians or healthcare professionals focus their time on reviewing higher quality images and/or assess the level of uncertainty of their review, and/or may be used to automatically decrease the number of images in a study.

In accordance with aspects of the present disclosure, a system for assessing gastrointestinal tract cleansing includes one or more processors and at least one memory. The memory stores instructions which, when executed by the one or more processors, cause the system to: access a plurality of images of at least a portion of a gastrointestinal tract (GIT) captured by a capsule endoscopy device; access a cleansing score for each image of the plurality of images; for each image of the plurality of images, access advancement information indicative of physical advancement of the capsule endoscopy device relating to the respective image; determine a cleansing measure for at least the portion of the GIT based on the cleansing score and the advancement information for each image of the plurality of images; and provide a visual indication based on at least one of: the cleansing measure for at least the portion of the GIT or at least one of the cleansing scores.

In various embodiments of the system, the plurality of images includes a first image and successive images, the advancement information for the first image of the plurality of images includes an estimate of how much the capsule endoscopy device has advanced from a beginning of the at least the portion of the GIT to reach a location where the first image was captured, and for each of the successive images of the plurality of images, the advancement information includes an estimate of how much the capsule endoscopy device has advanced from a location where an immediate prior image was captured to reach a location where that image was captured.

In various embodiments of the system, the at least the portion of the GIT is one of: an entirety of a GIT segment of interest or a portion of the GIT segment of interest. In various embodiments of the system, the GIT segment of interest is one of: a small bowel or a colon.

In various embodiments of the system, the cleansing measure for at least the portion of the GIT is computed as:

$$\frac{\sum_{i=1}^{n}(score_i \cdot \Delta_i)}{\sum_{i=1}^{n}\Delta_i}$$

where $n$ is a number of images in the plurality of images, $score_i$ is the cleansing score for image $i$, and $\Delta_i$ is the advancement information for image $i$.

In various embodiments of the system, $\sum_{i=1}^{n}\Delta_i$ is less than 1% of the entirety of the GIT segment of interest.

In various embodiments of the system, the instructions, when executed by the one or more processors, further cause the system to determine a cleansing measure for the entire GIT segment of interest based on the cleansing measure for at least the portion of the GIT.

In various embodiments of the system, the advancement information indicative of physical advancement of the capsule endoscopy device relating to an image is indicative of whether the capsule endoscopy device is static.

In various embodiments of the system, in determining the cleansing measure for at least the portion of the GIT, the instructions, when executed by the one or more processors, cause the system to exclude cleansing scores for images whose advancement information indicates that the capsule endoscopy device is static.

In various embodiments of the system, the instructions, when executed by the one or more processors, further cause the system to: for each image of the plurality of images, access wall assessment information indicating whether the image is an image of only a clean wall of the at least the portion of the GIT, and in determining the cleansing measure for at least the portion of the GIT, the instructions, when executed by the one or more processors, cause the system to exclude cleansing scores for images whose wall assessment information indicates that the image is an image of only a clean wall.

In various embodiments of the system, in determining the cleansing measure for at least the portion of the GIT, the instructions, when executed by the one or more processors, cause the system to compute the cleansing measure for at least the portion of the GIT as an average of the cleansing scores which were not excluded.

In various embodiments of the system, the at least the portion of the GIT is one of: a portion of a small bowel, an entirety of a small bowel, a portion of a colon, or an entirety of a colon.

In various embodiments of the system, the cleansing score for each image of the plurality of images is based on a predetermined set of scores including: a first score indicative of poor visualization of mucosa and indicative of at least one of: excessive content, excessive debris, excessive bubbles, excessive bile staining, excessive chyme staining, or excessive bleeding; a second score indicative of moderate visualization of mucosa and indicative at least one of: moderate content, moderate debris, moderate bubbles, moderate bile staining, moderate chyme staining, or blurriness; a third score indicative of good visualization of mucosa not through a big bubble and indicative at least one of: mild amount of debris, mild bubbles, mild bile staining, or mild chyme staining; and a fourth score indicative of excellent visualization of mucosa not through a big bubble and indicative at least one of: minimal amount of debris, minimal bubbles, minimal bile staining, minimal chyme staining, no debris, no bubbles, no bile staining, or no chyme staining.

In various embodiments of the system, the cleansing score for each image of the plurality of images is based on a predetermined set of scores including: a first score indicative of large amounts of fecal and bubble content that significantly interfere with mucosal visualization; a second score indicative of moderate amounts of fecal and bubble content that substantially interfere with mucosal visualization; a third score indicative of mild to moderate amounts of fecal and bubble content that moderately interfere with mucosal visualization; and a fourth score indicative of mild amounts of fecal and bubble content that do not interfere with mucosal visualization.

In accordance with aspects of the present disclosure, a computer-implemented method for assessing gastrointestinal tract cleansing includes: accessing a plurality of images of at least a portion of a gastrointestinal tract (GIT) captured by a capsule endoscopy device; accessing a cleansing score for each image of the plurality of images; for each image of the plurality of images, accessing advancement information indicative of physical advancement of the capsule endoscopy device relating to the respective image; determining a cleansing measure for at least the portion of the GIT based on the cleansing score and the advancement information for each image of the plurality of images; and providing a visual indication based on at least one of: the cleansing measure for at least the portion of the GIT or at least one of the cleansing scores.

In various embodiments of the computer-implemented method, the plurality of images includes a first image and successive images, the advancement information for the first image of the plurality of images includes an estimate of how much the capsule endoscopy device has advanced from a beginning of the at least the portion of the GIT to reach a location where the first image was captured, and for each of the successive images of the plurality of images, the advancement information includes an estimate of how much the capsule endoscopy device has advanced from a location where an immediate prior image was captured to reach a location where that image was captured.

In various embodiments of the computer-implemented method, the at least the portion of the GIT is one of: an entirety of a GIT segment of interest or a portion of the GIT segment of interest. In various embodiments of the computer-implemented method, the GIT segment of interest is one of: a small bowel or a colon.

In various embodiments of the computer-implemented method, the cleansing measure for at least the portion of the GIT is computed as:

$$\frac{\sum_{i=1}^{n}(score_i \cdot \Delta_i)}{\sum_{i=1}^{n} \Delta_i}$$

where $n$ is a number of images in the plurality of images, $score_i$ is the cleansing score for image $i$, and $\Delta_i$ is the advancement information for image $i$.

In various embodiments of the computer-implemented method, $\sum_{i=1}^{n} \Delta_i$ is less than 1% of the entirety of the GIT segment of interest.

In various embodiments of the computer-implemented method, the computer-implemented method includes determining a cleansing measure for the entire GIT segment of interest based on the cleansing measure for at least the portion of the GIT.

In various embodiments of the computer-implemented method, the advancement information indicative of physical advancement of the capsule endoscopy device relating to an image is indicative of whether the capsule endoscopy device is static.

In various embodiments of the computer-implemented method, determining the cleansing measure for at least the portion of the GIT involves excluding cleansing scores for images whose advancement information indicates that the capsule endoscopy device is static.

In various embodiments of the computer-implemented method, the computer-implemented method includes, for each image of the plurality of images, accessing wall assessment information indicating whether the image is an image of only a clean wall of the at least the portion of the GIT, and determining the cleansing measure for at least the portion of the GIT involves excluding cleansing scores for images whose wall assessment information indicates that the image is an image of only a clean wall.

In various embodiments of the computer-implemented method, determining the cleansing measure for at least the portion of the GIT includes computing the cleansing measure for at least the portion of the GIT as an average of the cleansing scores which were not excluded.

In various embodiments of the computer-implemented method, the at least the portion of the GIT is one of: a portion of a small bowel, an entirety of a small bowel, a portion of a colon, or an entirety of a colon.

In various embodiments of the computer-implemented method, the cleansing score for each image of the plurality of images is based on a predetermined set of scores including: a first score indicative of poor visualization of mucosa and indicative of at least one of: excessive content, excessive debris, excessive bubbles, excessive bile staining, excessive chyme staining, or excessive bleeding; a second score indicative of moderate visualization of mucosa and indicative at least one of: moderate content, moderate debris, moderate bubbles, moderate bile staining, moderate chyme staining, or blurriness; a third score indicative of good visualization of mucosa not through a big bubble and indicative at least one of: mild amount of debris, mild bubbles, mild bile staining, or mild chyme staining; and a fourth score indicative of excellent visualization of mucosa not through a big bubble and indicative at least one of: minimal amount of debris, minimal bubbles, minimal bile staining, minimal chyme staining, no debris, no bubbles, no bile staining, or no chyme staining.

In various embodiments of the computer-implemented method, the cleansing score for each image of the plurality of images is based on a predetermined set of scores that include: a first score indicative of large amounts of fecal and bubble content that significantly interfere with mucosal visualization; a second score indicative of moderate amounts of fecal and bubble content that substantially interfere with mucosal visualization; a third score indicative of mild to moderate amounts of fecal and bubble content that moderately interfere with mucosal visualization; and a fourth score indicative of mild amounts of fecal and bubble content that do not interfere with mucosal visualization.

In accordance with aspects of the present disclosure, a computer-readable medium stores instructions. The instructions, when executed by a computer, causes the computer to perform a method that includes: accessing a plurality of images of at least a portion of a gastrointestinal tract (GIT) captured by a capsule endoscopy device; accessing a cleansing score for each image of the plurality of images; for each image of the plurality of images, accessing advancement information indicative of physical advancement of the capsule endoscopy device relating to the respective image; determining a cleansing measure for at least the portion of the GIT based on the cleansing score and the advancement information for each image of the plurality of images; and providing a visual indication based on at least one of: the cleansing measure for at least the portion of the GIT or at least one of the cleansing scores.

Further details and aspects of exemplary embodiments of the present disclosure are described in more detail below with reference to the appended figures.

BRIEF DESCRIPTION OF DRAWINGS

The above and other aspects and features of the disclosure will become more apparent in view of the following detailed description when taken in conjunction with the accompanying drawings wherein like reference numerals identify similar or identical elements.

FIG. 1 is a diagram illustrating an exemplary gastrointestinal tract (GIT);
FIG. 2 is a block diagram of an exemplary system for capturing images in vivo via a Capsule Endoscopy (CE) procedure and for processing the captured images, in accordance with aspects of the present disclosure;
FIG. 3 is a block diagram of an exemplary computing system which may be used with the systems of the present disclosure;
FIG. 4 is a diagram of a portion of a GIT or the entire GIT, and of a corresponding stream of images captured within the GIT, in accordance with aspects of the present disclosure;
FIG. 5 is a block diagram of an exemplary machine learning system, in accordance with aspects of the present disclosure;
FIG. 6 is a block diagram of exemplary data associated with an image, in accordance with aspects of the present disclosure; and
FIG. 7 is a flow diagram of an exemplary operation for determining a cleansing measure for a portion of a GIT, for a GIT segment of interest, or for the entirety of a GIT, in accordance with aspects of the present disclosure.

DETAILED DESCRIPTION

The present disclosure relates to systems and methods for processing images to assess a degree of cleansing of a gastrointestinal tract (GIT) or of portions of a GIT. Indicating the degree of cleansing of a GIT or of portions of a GIT may help physicians or healthcare professionals focus their time on reviewing higher quality images and/or assess the level of uncertainty in their review, and/or may be used to automatically decrease the number of images in a study.

In the following detailed description, specific details are set forth in order to provide a thorough understanding of the disclosure. However, it will be understood by those skilled in the art that the disclosure may be practiced without these specific details. In other instances, well-known methods, procedures, and components have not been described in detail so as not to obscure the present disclosure. Some features or elements described with respect to one system may be combined with features or elements described with respect to other systems. For the sake of clarity, discussion of same or similar features or elements may not be repeated.

Although the disclosure is not limited in this regard, discussions utilizing terms such as, for example, "processing," "computing," "calculating," "determining," "establishing," "analyzing," "checking," or the like, may refer to operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium that may store instructions to perform operations and/or processes. Although the disclosure is not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. The term set when used herein may include one or more items. Unless explicitly stated, the methods described herein are not constrained to a particular order or sequence. Additionally, some of the described methods or elements thereof can occur or be performed simultaneously, at the same point in time, or concurrently.

The term "location" and its derivatives, as referred to herein with respect to an image, may refer to the estimated location of the capsule along the GIT while capturing the image or to the estimated location of the portion of the GIT shown in the image along the GIT.

A type of CE procedure may be determined based on, inter alia, the portion of the GIT that is of interest and is to be imaged (e.g., the colon or the small bowel ("SB")), or based on the specific use (e.g., for checking the status of a GI disease, such as Crohn's disease, or for colon cancer screening).

The terms screen(s), view(s) and display(s) may be used herein interchangeably and may be understood according to the specific context.

The terms "surrounding" or "adjacent" as referred to herein with respect to images (e.g., images that surround another image(s), or that are adjacent to other image(s)), may relate to spatial and/or temporal characteristics unless specifically indicated otherwise. For example, images that surround or are adjacent to other image(s) may be images that are estimated to be located near the other image(s) along the GIT and/or images that were captured near the capture time of another image, within a certain threshold, e.g., within one or two centimeters, or within one, five, or ten seconds.

The terms "GIT" and "a portion of the GIT" may each refer to or include the other, according to their context. Thus, the term "a portion of the GIT" may also refer to the entire GIT and the term "GIT" may also refer only to a portion of the GIT.

The terms "image" and "frame" may each refer to or include the other and may be used interchangeably in the present disclosure to refer to a single capture by an imaging device. For convenience, the term "image" may be used more frequently in the present disclosure, but it will be understood that references to an image shall apply to a frame as well.

The term "classification score(s)" or "score(s)" may be used throughout the specification to indicate a value or a vector of values for a category or a set of categories applicable to an image/frame. In various implementations, the value or vector of values of a classification score or classification scores may be or may reflect probabilities. In various embodiments, a model may output classification scores which may be probabilities. In various embodiments, a model may output classification scores which may not be probabilities.

The term "classification probabilities" may be used to describe classification scores which are probabilities or to describe a transformation of classification scores which are not probabilities into values which reflect the probabilities that each category of the set of categories applies to the image/frame. It will be understood from context that various references to "probability" refer to and are a shorthand for a classification probability.

As used herein, a "machine learning system" means and includes any computing system that implements any type of machine learning. As used herein, "deep learning neural network" refers to and includes a neural network having several hidden layers and which does not require feature selection or feature engineering. A "classical" machine learning system, in contrast, is a machine learning system which requires feature selection or feature engineering.

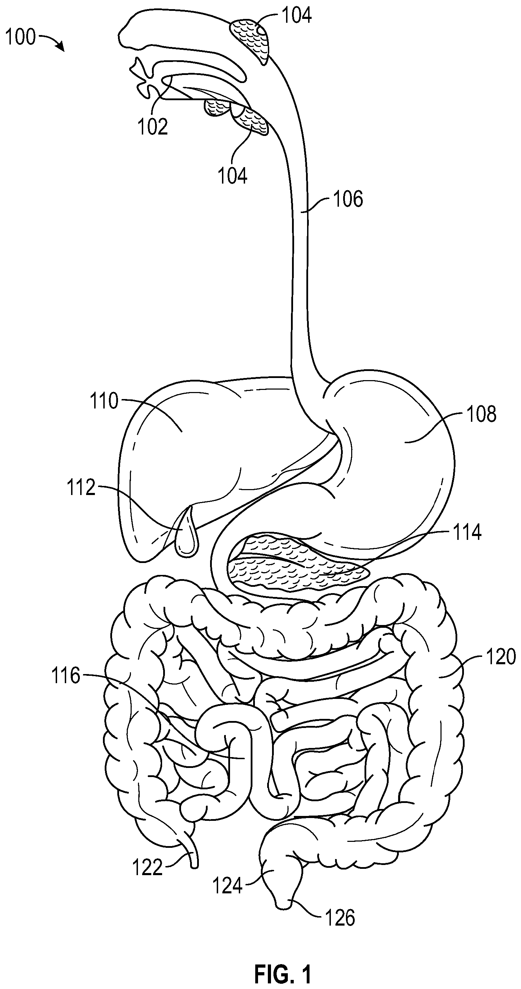

Referring to FIG. 1, an illustration of the GIT 100 is shown. The GIT 100 is an organ system within humans and other animals. The GIT 100 generally includes a mouth 102 for taking in sustenance, salivary glands 104 for producing saliva, an esophagus 106 through which food passes aided by contractions, a stomach 108 to secret enzymes and stomach acid to aid in digesting food, a liver 110, a gall bladder 112, a pancreas 114, a small intestine/small bowel 116 ("SB") for the absorption of nutrients, and a colon 120 (e.g., large intestine) for storing water and waste material as feces prior to defecation. The colon 120 includes an appendix 122, a rectum 124, and an anus 126, among other portions. The colon 120 may be divided, for example, into five anatomical segments: cecum, right or ascending colon, transverse colon, left or descending colon (e.g., left colon-sigmoid), and rectum 124. An ileum is the final section of the small bowel 116. The cecum is the first section of the colon 120. The ileum and the cecum are separated by a muscle valve called the ileocecal valve (ICV). The rectum 126 is the last section of the colon 120. Between the cecum and the rectum 126 are the right or ascending colon, the transverse colon, and the left or descending colon (e.g., left colon-sigmoid). Food taken in through the mouth is digested by the GIT to take in nutrients and the remaining waste is expelled as feces through the anus 126.

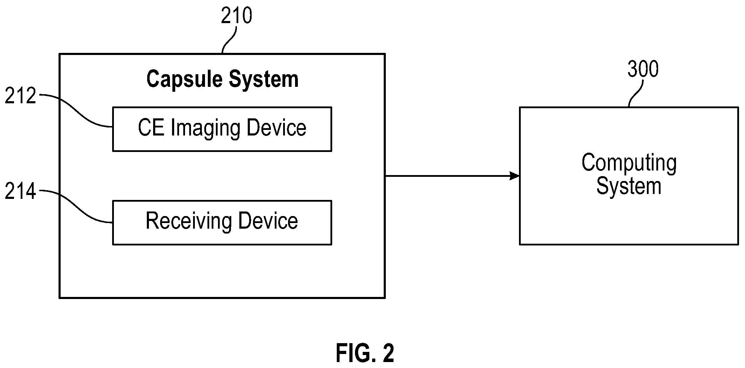

A CE imaging device (e.g., 212, FIG. 2) may be used to image the interior of the small bowel 116 and the colon 120. The small bowel 116 is narrower than the colon 120 and, in the small bowel 116, a CE imaging device generally proceeds unidirectionally and does not move backwards. The entrance from the small bowel 116 into the colon 120 occurs through the ICV. Usually after entering the colon 120 through the ICV, a CE imaging device goes into the cecum. However, occasionally, a CE imaging device may miss the cecum and go straight into the ascending colon. The colon 120 may be wide enough to enable almost unrestricted CE imaging device movement. For example, in the colon, an CE imaging device may rotate and roll, may move backwards, may rest in one place for a long period of time, and/or may move very fast through various portions.

Studies of different portions of the GIT 100 (e.g., colon 120, small bowel 116, and/or stomach 108) may be presented via a suitable user interface. As used herein, the term "study" refers to and includes at least a set of images selected from the images captured by a CE imaging device (e.g., 212, FIG. 2) during a single CE procedure performed with respect to a specific patient and at a specific time, and can optionally include information other than images as well. The type of procedure performed may determine which portion of the GIT 100 is the portion of interest. Examples of types of procedures performed include, without limitation, a small bowel procedure, a colon procedure, a small bowel and colon procedure, a procedure aimed to specifically exhibit or check the small bowel, a procedure aimed to specifically exhibit or check the colon, a procedure aimed to specifically exhibit or check the colon and the small bowel, or a procedure to exhibit or check the entire GIT: esophagus, stomach, SB, and colon.

FIG. 2 shows a block diagram of a system for analyzing medical images captured in vivo via a CE procedure. The system generally includes a capsule system 210 configured to capture images of the GIT and a computing system 300 (e.g., local system and/or cloud system) configured to process the captured images.

The capsule system 210 may include a swallowable CE imaging device 212 (e.g., a capsule) configured to capture images of the GIT as the CE imaging device 212 travels through the GIT. In various embodiments, the CE imaging device 212 may have a single imaging sensor. In various embodiments, the CE imaging device 212 may have more than one imaging sensor, such as two imaging sensors. For example, the CE imaging device 212 may have the form of a capsule and both ends of the capsule may have an imaging sensor. The images captured by the CE imaging device 212 may be stored on the CE imaging device 212 and/or transmitted to a receiving device 214 typically including an antenna. In various embodiments involving multiple imaging sensors, the images captured by each imaging sensor may be identified and may be distinguished from images captured by any other imaging sensor. In some capsule systems 210, the receiving device 214 may be located on the patient who swallowed the CE imaging device 212 and may, for example, take the form of a belt worn by the patient or a patch secured to the patient.

The capsule system 210 may be communicatively coupled with the computing system 300 and can communicate captured images to the computing system 300. The computing system 300 may process the received images using image processing technologies, machine learning technologies, and/or signal processing technologies, among other technologies. The computing system 300 can include local computing devices that are local to the patient and/or the patient's treatment facility, a cloud computing platform that is provided by cloud services, or a combination of local computing devices and a cloud computing platform.

In the case where the computing system 300 includes a cloud computing platform, the images captured by the capsule system 210 may be transmitted online to the cloud computing platform. In various embodiments, the images can be transmitted via the receiving device 214 worn or carried by the patient. In various embodiments, the images can be transmitted via the patient's smartphone or via any other device connected to the Internet and which may be coupled with the CE imaging device 212 or the receiving device 214.

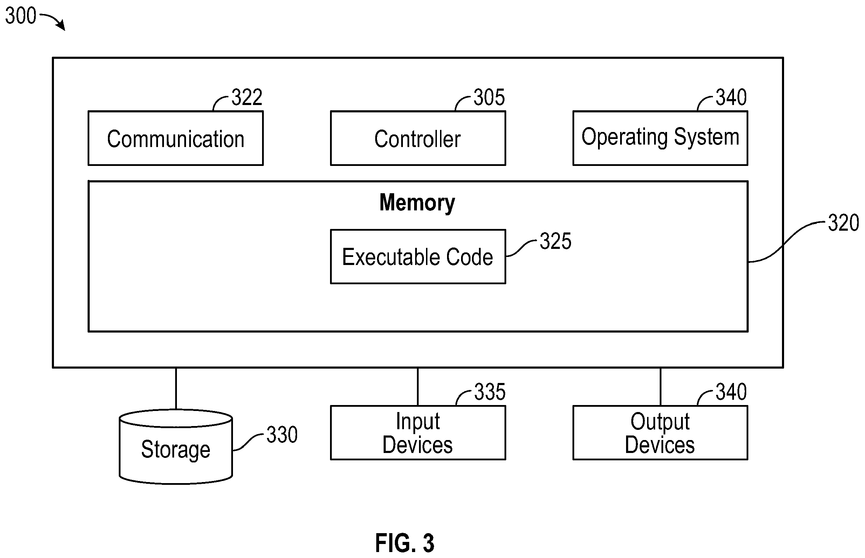

FIG. 3 shows a high-level block diagram of an exemplary computing system 300 that may be used with image analyzing systems of the present disclosure. Computing system 300 may include a processor or controller 305 that may be or include, for example, one or more central processing unit processor(s) (CPU), one or more Graphics Processing Unit(s) (GPU or GPGPU), a chip or any suitable computing or computational device, an operating system 215, a memory 320, a storage 330, input devices 335 and output devices 340. Modules or equipment for collecting or receiving (e.g., a receiver worn on a patient) or displaying or selecting for display (e.g., a workstation) medical images collected by the CE imaging device 212 (FIG. 2) may be or include, or may be executed by, the computing system 300 shown in FIG. 3. A communication component 322 of the computing system 300 may allow communications with remote or external devices, e.g., via the Internet or another network, via radio, or via a suitable network protocol such as File Transfer Protocol (FTP), etc.

The computing system 300 includes an operating system 315 that may be or may include any code segment designed and/or configured to perform tasks involving coordination, scheduling, arbitration, supervising, controlling or otherwise managing operation of computing system 300, for example, scheduling execution of programs. Memory 320 may be or may include, for example, a Random Access Memory (RAM), a read-only memory (ROM), a Dynamic RAM (DRAM), a Synchronous DRAM (SD-RAM), a double data rate (DDR) memory chip, a Flash memory, a volatile memory, a nonvolatile memory, a cache memory, a buffer, a short term memory unit, a long term memory unit, or other suitable memory units or storage units. Memory 320 may be or may include a plurality of possibly different memory units. Memory 320 may store for example, instructions to carry out a method (e.g., executable code 325), and/or data such as user responses, interruptions, etc.

Executable code 325 may be any executable code, e.g., an application, a program, a process, task, or script. Executable code 325 may be executed by controller 305 possibly under control of operating system 315. For example, execution of executable code 325 may cause the display or selection for display of medical images as described herein. In some systems, more than one computing system 300 or components of computing system 300 may be used for multiple functions described herein. For the various modules and functions described herein, one or more computing systems 300 or components of computing system 300 may be used. Devices that include components similar or different to those included in the computing system 300 may be used and may be connected to a network and used as a system. One or more processor(s) 305 may be configured to carry out methods of the present disclosure by for example executing software or code. Storage 330 may be or may include, for example, a hard disk drive, a floppy disk drive, a Compact Disk (CD) drive, a CD-Recordable (CD-R) drive, a universal serial bus (USB) device or other suitable removable and/or fixed storage unit. Data such as instructions, code, medical images, image streams, etc. may be stored in storage 330 and may be loaded from storage 330 into memory 320 where it may be processed by controller 305. In some embodiments, some of the components shown in FIG. 3 may be omitted.

Input devices 335 may include for example a mouse, a keyboard, a touch screen or pad or any suitable input device. It will be recognized that any suitable number of input devices may be operatively coupled to computing system 300. Output devices 340 may include one or more monitors, screens, displays, speakers and/or any other suitable output devices. It will be recognized that any suitable number of output devices may be operatively coupled to computing system 300 as shown by block 340. Any applicable input/output (I/O) devices may be operatively coupled to computing system 300, for example, a wired or wireless network interface card (NIC), a modem, printer or facsimile machine, a universal serial bus (USB) device or external hard drive may be included in input devices 335 and/or output devices 340.

Multiple computer systems 300 including some or all of the components shown in FIG. 3 may be used with the described systems and methods. For example, a CE imaging device 212, a receiver, a cloud-based system, and/or a workstation or portable computing device for displaying images may include some or all of the components of the computer system of FIG. 3. A cloud platform (e.g., a remote server) including components such as computing system 300 of FIG. 3 may receive procedure data such as images and metadata, processes and generate a study, and may also display the generated study for the doctor's review (e.g., on a web browser executed on a workstation or portable computer). An "on-premises" option, may use a workstation or local server of a medical facility to store, process and display images and/or a study.

According to some aspects of the present disclosure, a user (e.g., a physician), may build his or her understanding of a case by reviewing a study, e.g., a display of images (e.g., captured by the CE imaging device 212) that were selected, e.g., automatically, as images that may be of interest. In some systems of the present disclosure, a relatively small number of images from the captured images are displayed for the user's review per case. By "relatively small number" it is meant on the order of hundreds at most or at least at average as opposed to current methods, which display a video stream of images that typically includes thousands of images per a case (e.g., around 6,000 images). In some systems, only up to a few hundreds of images are displayed for the user's review. In some systems, the number of images displayed for the user's review is up to an order of 1,000. Browsing through a relatively small number of images, as opposed to watching or reviewing thousands of images, may significantly ease the review process for the user, reduce the reading time per case and may lead to better diagnosis. Aspects of exemplary user interfaces for displaying a study are described in co-pending International Patent Application Publication No. WO/2020/079696, entitled "Systems and Methods for Generating and Displaying a Study of a Stream of In-Vivo Images". Other aspects of the computing system 300 and the capsule system (210, FIG. 2) are described in co-pending U.S. Provisional Application No. 62/867,050, entitled "Systems and Methods For Capsule Endoscopy Procedure".

The following description may focus on images of a small bowel (SB) or images of a colon captured by a capsule endoscopy device. Such SB images or colon images may be part of a stream of images of the GIT in the manner described below or by using other methodologies which persons skilled in the art would recognize. SB images and colon images are used merely as an example of the aspects and embodiments described below. The embodiments and aspects described herein also apply to other portions of a GIT, and it is intended that any description related to SB images or colon images shall be applicable to images of other portions of a GIT.

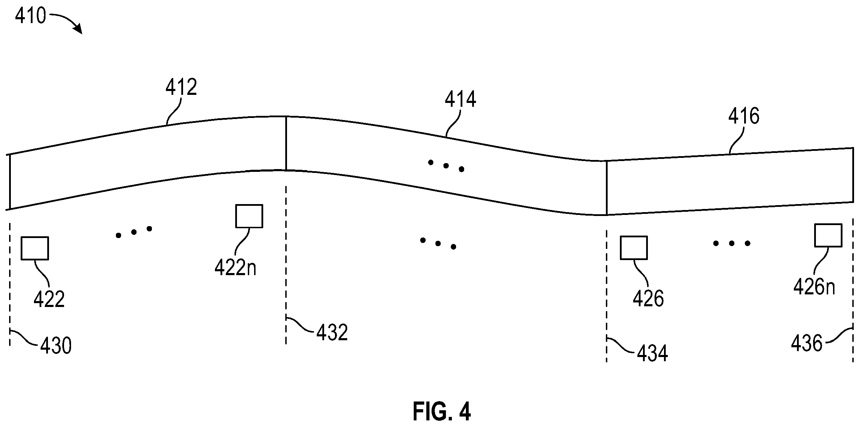

As mentioned above, a CE imaging device (e.g., 212, FIG. 2) captures images of a GIT as it progresses through a GIT. FIG. 4 shows a diagram of a portion of a GIT 410 or an entirety of a GIT 410 and of images 422-426n captured by a CE imaging device within the portion or the entirety of the GIT 410. For example, the illustration of FIG. 4 may represent the entirety of the GIT 410, where the first portion 412 may be pre-small bowel, the middle portion 414 may be the small bowel, and the last portion 416 may be the colon. If the illustration of FIG. 4 represents a portion of the GIT 410 such as the small bowel, then the numerals 412, 414, 416 may correspond to portions of the small bowel. Any number of portions 412-416 may be used in accordance with the present disclosure, such as, for example, five portions, one hundred portions or one-thousand portions of the small bowel or another number. If the illustration of FIG. 4 represents a portion of the GIT 410 such as the colon, then the numerals 412-416 may correspond to portions of the colon. As described above, the colon has five main portions, i.e., cecum, right or ascending colon, transverse colon, left or descending colon, and rectum. In various embodiments, another number of portions may be used, such as one hundred portions or one thousand portions of the colon or another number.

The stream of images 422-426n captured by a CE imaging device generally does not include location information to indicate the portion of the GIT in which the image was captured. Dividing a stream of GIT images into GIT portions (e.g., pre-small bowel, small bowel, and colon) is described in a U.S. Patent Application No. 63/018,890. Thus, if the illustration of FIG. 4 represents the entirety of a GIT, the dividing point in the stream of images between pre-small bowel and small bowel may correspond to point 432, and the dividing point in the stream of images between small bowel and colon may correspond to point 434. The beginning of the stream of images may be designated as a starting point 430, and the end of the stream of images may be designated as an ending point 436.

As another example, dividing a stream of colon images into colon portions (e.g., cecum, right or ascending colon, transverse colon, left or descending colon, and rectum) is described in U.S. Patent Application No. 17/244,988. Thus, if the illustration of FIG. 4 represents the colon, point 430 may correspond to the starting point of the cecum, point 432 may correspond to the dividing point between cecum and the right or ascending colon, and point 434 may correspond to the dividing point between left or descending colon and the rectum, and point 436 may correspond to the ending point of the rectum. The other dividing points at the beginning and at the end of the transverse colon may be similarly identified.

Persons skilled in the art will recognize other techniques for dividing a stream of GIT images into GIT portions, and such techniques are contemplated to be within the scope of the present disclosure. For example, if the illustration of FIG. 4 represents the small bowel, the stream of images may be divided into portions of approximately equal SB length using, for example, techniques described in the U.S. Patent Application Publication No. 2019/0244351. Such and other techniques are contemplated to be within the scope of the present disclosure.

With continuing reference to FIG. 4, each portion of the GIT shown in FIG. 4 has corresponding images from the stream of images 422-426n. For example, images 422-422n are images from the stream of images 422-426n which correspond to GIT portion 412, and images 426-426n are images from the stream of images 422-426n which correspond to GIT portion 416. Each portion may have any number of images and may have a same number or a different number of images as other portions.

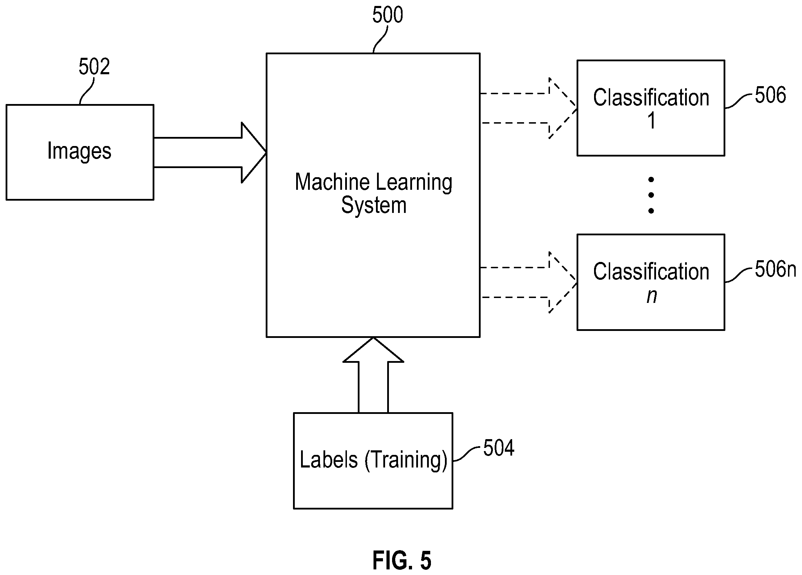

As explained in more detail below in connection with FIG. 5, a machine learning system may be used to classify images to indicate a cleansing score reflected in each image. In the stream of images 422-426n of FIG. 4, each image captured by a CE imaging device (e.g., 212, FIG. 2) can be communicated to a machine learning system, and the machine learning system can process the images 422-426n to determine a cleansing score for some or all of the images 422-426n. In accordance with aspects of the present disclosure, and as described in more detail below, a cleansing measure may be determined for each GIT portion (e.g., 412, 416) based on the cleansing scores for individual images and based on advancement information for each image. Advancement information will be described in more detail below. For now, it is sufficient to note that advancement information for an image may include, for example, information about whether the CE imaging device which captured the image was static or in motion and/or information about how much the CE imaging device advanced in the GIT between successive images.

With reference to FIG. 5, a block diagram of an exemplary machine learning system 500 for classifying images is shown. In various embodiments, the images 502 may include SB images, colon images, and/or images of another portion of a GIT, such as the images 422-426n shown in FIG. 4. In various embodiments, the images 502 may be images of one or more portions of a GIT. The machine learning system 500 operates to classify the input images 502, captured by the CE imaging device 212 (see FIG. 2), to one or more classifications 506-506n, such as classifications relating to degree of cleansing reflected in the image. The arrows leading to the classifications 506-506n are shown with dashed lines to indicate that each classification may or may not be chosen by the machine learning system 500 as being applicable to an input image 502. The machine learning system 500 may be executed on the computer system 300 (FIG. 3). Persons skilled in the art will understand the machine learning system 500 and how to implement it. For example, the machine learning system may include various types of deep learning neural networks, such as, without limitation, MobileNet or Inception.

The machine learning system 500 may be trained based on labeled training images. For example, a training image may be associated with a label 504 which indicates the degree of cleansing reflected in the image. Persons skilled in the art will understand training of a machine learning system 500 and how to implement the training, including manual labeling of training images by persons skilled in evaluating gastrointestinal cleansing. In accordance with aspects of the present disclosure, the degree of cleaning reflected in the training images may be manually labeled by skilled persons based on a predetermined set of degrees and/or scores.

As an example, in various embodiments, a predetermined set of degrees and/or scores indicative of small bowel cleansing can include: a first degree or score (e.g., poor, or score "1") indicative of poor visualization of mucosa and indicative of at least one of: excessive content, excessive debris, excessive bubbles, excessive bile staining, excessive chyme staining, or excessive bleeding; a second degree or score (e.g., fair, or score "2") indicative of moderate visualization of mucosa and indicative at least one of: moderate content, moderate debris, moderate bubbles, moderate bile staining, moderate chyme staining, or blurriness; a third degree or score (e.g., good, or score "3") indicative of good visualization of mucosa not through a big bubble and indicative at least one of: mild amount of debris, mild bubbles, mild bile staining, or mild chyme staining; and a fourth degree or score (e.g., excellent, or score "4") indicative of excellent visualization of mucosa not through a big bubble and indicative at least one of: minimal amount of debris, minimal bubbles, minimal bile staining, minimal chyme staining, no debris, no bubbles, no bile staining, or no chyme staining. These predetermined set of degrees and/or scores may also be the classifications (e.g., 506-506n) used by the machine learning system 500 to classify the input images 502. For example, the machine learning system 500 may classify each input image 502 as having poor cleansing/score "1", fair cleansing/score "2", good cleansing/score "3", or excellent cleansing/score "4". In various embodiments, the predetermined set of degrees and/or scores may be used to train and classify small bowel images and/or other images of the gastrointestinal tract.

In various embodiments, and as another example, a predetermined set of degrees and/or scores indicative of colon cleansing can include: a first degree or score (e.g., poor, or score "1") indicative of large amounts of fecal and bubble content that significantly interfere with mucosal visualization; a second degree or score (e.g., fair, or score "2") indicative of moderate amounts of fecal and bubble content that substantially interfere with mucosal visualization; a third degree or score (e.g., good, or score "3") indicative of mild to moderate amounts of fecal and bubble content that moderately interfere with mucosal visualization; and a fourth degree or score (e.g., excellent, or score "4") indicative of mild amounts of fecal and bubble content that do not interfere with mucosal visualization. The foregoing cleansing definitions are different from prior cleansing definitions in that they do not depend on characteristics which refer to visualization, and they use flexible terms rather than specific criteria such as percentage levels. The foregoing cleansing definitions are easier for physicians and other medical professional to recognize and apply consistently. These predetermined set of degrees and/or scores may be used to label training images and may also be the classifications (e.g., 506-506n) used by the machine learning system 500 to classify the input images 502. For example, the machine learning system 500 may classify each input image 502 as having poor cleansing/score "1", fair cleansing/score "2", good cleansing/score "3", or excellent cleansing/score "4". In various embodiments, the predetermined set of degrees and/or scores may be used to train and classify colon images and/or other images of the gastrointestinal tract. The predetermined set of degrees and/or scores described above are exemplary, and other degrees and/or scores and other cleansing definitions are contemplated to be within the scope of the present disclosure.

As persons skilled in the art will understand, certain types of machine learning systems may provide multiple classification probabilities which indicate the probability that each classification 506-506n applies to the input images 502, and certain types of machine learning systems may apply regression techniques to provide values of a continuous measure that indicates which of the classifications 506-506n is most applicable to each input image 502. Both of these configurations are described in more detail below in connection with providing a cleansing score for each image in a stream of images of the GIT, such as the stream of images 422-426n shown in FIG. 4. Such configurations and types of machine learning systems are exemplary, and other types of machine learning models and techniques may be used for the machine learning system 500 of FIG. 5.

With reference to FIG. 4 and FIG. 5, a cleansing score may be provided for each image in the stream of images 422-426n of FIG. 4 using the machine learning system 500 of FIG. 5. As mentioned above, the classifications 506-506n may include four cleansing classifications: poor cleansing/score "1", fair cleansing/score "2", good cleansing/score "3", or excellent cleansing/score "4". However, other numbers of cleansing classifications may be used and are within the scope of the present disclosure. Generally, there may be a number N of cleansing classifications. For example, score "1" may correspond to the dirtiest cleansing classification, score N may correspond to the cleanest cleansing classification, and scores "2" through N-1 may correspond to varying degrees of cleansing in between. The following description may refer to cleansing classification and clean score interchangeably, such that a reference to a particular cleansing score (e.g., score "1") will be understood to refer to a particular cleansing classification, and vice versa.

**In** accordance with aspects of the present disclosure, the machine learning system 500 may provide classification probabilities which indicate the probability that each of N cleansing classifications/scores applies to an input image. For N cleansing scores, the machine learning system 500 may provide $N$ probabilities $P_{score\_1} \cdot \cdot \cdot P_{score\_N}$, such that $\sum_{i=1}^{N} P_i = 1$. In accordance with aspects of the present disclosure, in such a machine learning system, the cleansing score for an image may be determined as an expected value:

$$\sum_{i=1}^{N}(P_i \cdot score_i)$$

. In various embodiments, $score_i = i$, i.e., $score_i = 1$, $score_2 = 2$, ..., $score_N = N$. In various embodiments, each $score_i$ may have any value. Accordingly, the machine learning system 500 may process each image in the stream of images 422-426n to provide classification probabilities, and such classification probabilities may be used to provide a cleansing score that is an expected value.

In accordance with aspects of the present disclosure, the machine learning system 500 may apply regression techniques to provide values of a continuous cleansing score that indicates which of the classifications 506-506n is most applicable to an input image. In such a machine learning system, rather than provide a cleansing probability for each of $N$ cleansing classifications, the machine learning system provides a value in a continuous range for each input image. For example, if the lowest cleansing classification has $score_1$ and the highest cleansing classification has score $score_N$, the continuous range may be [$score_1$, $score_N$]. Using a numerical example, if the lowest cleansing classification has score "1" and the highest cleansing classification as score "4", the cleansing scoring may be a value in the continuous range [1, 4]. Because the cleansing score is a value in a continuous range, the continuous range may map to any number of classifications 506-506n. For example, the continuous range may map to four cleansing classifications, e.g., poor, fair, good, and excellent. In various embodiments, the continuous range may map to any number of cleansing classifications, and such embodiments are contemplated to be within the scope of the present disclosure.

In accordance with aspects of the present disclosure, and with continuing reference to FIG. 5, additional instances of the machine learning system 500 may be implemented for various purposes. As described in more detail below, a machine learning system may be implemented to classify input images 502 to indicate whether or not the image includes only a clean gastrointestinal wall. In various embodiments, images of only a clean gastrointestinal wall may be too focused and may not provide sufficient surface area to accurately determine a degree of cleansing, and as described below, such images may be excluded from consideration.

The embodiment illustrated in FIG. 5 and the embodiments described in connection with FIG. 5 are exemplary, and other ways of classifying images are contemplated to be within the scope of the present disclosure. For example, in various embodiments, unsupervised learning or another type of learning may be used. In various embodiments, the classification can be performed by various configurations or combinations of deep learning neural networks, classical machine learning systems, and/or by machine learning models and techniques which persons skilled in the art will recognize. Such variations are contemplated to be within the scope of the present disclosure.

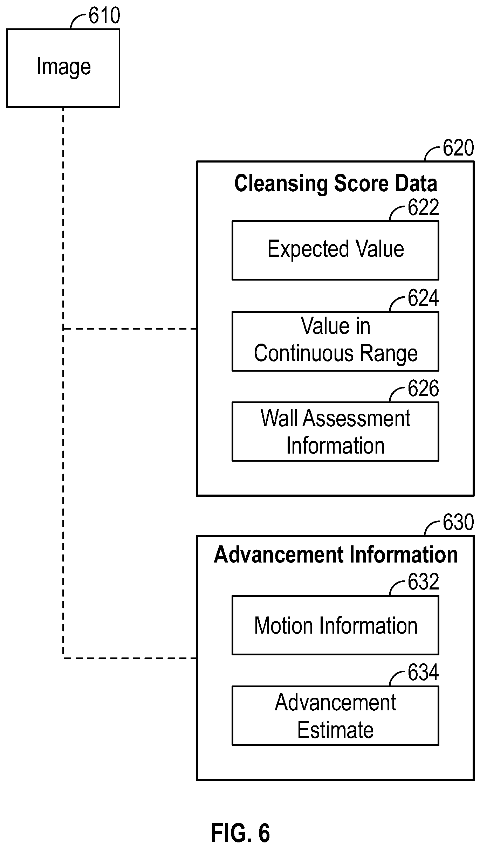

FIG. 6 shows a diagram of exemplary data that is associated with an image, such as any image in the image stream 422-426n of FIG. 4. In accordance with aspects of the present disclosure, the data associated with an image includes cleansing score data 620 and advancement information 630. The cleansing score data 620 may include a cleansing score expected value 622 (

$$\sum_{i=1}^{N}(P_i \cdot score_i)$$

) as described above in connection with FIG. 5 and/or may include a cleansing score value in a continuous range [$score_1$, $score_N$] 624 as described above in connection with FIG. 5. In various embodiments, as mentioned above, the cleansing score data 620 may include wall assessment information 626 which indicates whether or not the image 610 includes only a clean gastrointestinal wall. In various embodiments, images of only a clean gastrointestinal wall may be too focused and may not provide sufficient surface area to accurately determine a degree of cleansing. As such, an image of only a clean gastrointestinal wall may be excluded from consideration, and the cleansing score for such an image may be excluded from consideration. In various embodiments, the cleansing score data 620 may include all three of the illustrated data 622-626 or may include fewer than all three. The illustrated data is exemplary and the cleansing score data 620 may include other data related to cleansing scores.

The advancement information 630 for an image 610 may include motion information 632 which indicates whether the CE imaging device which captured the image was static or in motion and/or may include an estimate 634 of how much the CE imaging device advanced in the GIT between successive images to reach the location where the image 610 was captured. If the image 610 is the first captured image, the advancement information 634 may include an estimate of how much the CE imaging device advanced from a starting point to reach the location where the first image was captured. The advancement estimate 634 may have units (e.g., millimeters) or may not have any units. In various embodiments, the advancement estimate 634 may be expressed as a percentage of the total GIT or a percentage of a GIT segment of interest. Persons skilled in the art will recognize techniques for estimating whether a CE imaging device (e.g., 212, FIG. 2) which captured an image was static or in motion, and will recognize techniques for estimating how much a CE imaging device advanced between successive images. Such techniques may include, for example, the techniques described in U.S. Patent No. 8,792,691. Such and other techniques are contemplated to be within the scope of the present disclosure for determining the motion information 632 and/or the advancement estimate 634. In various embodiments, the advancement information 630 may include both of the illustrated data 632, 634 or may include just one of the illustrated data 632, 634. The illustrated data is exemplary and the advancement information 630 may include other data related to advancement of a CE imaging device.

The cleansing score data 620 and the advancement information 630 associated with an image 610 may be provided by a computing system, such as the computing system 300 of FIG. 3, and/or by a machine learning system, such as the machine learning system 500 of FIG. 5. In various embodiments, the motion information 632 may be provided by a capsule system, such as the capsule system 210 of FIG. 2. The following paragraphs will describe use of the cleansing score data 620 and the advancement information 630 associated with an image 610 to provide a cleansing measure for a portion of a GIT, for a GIT segment of interest, and/or for the entirety of the GIT.

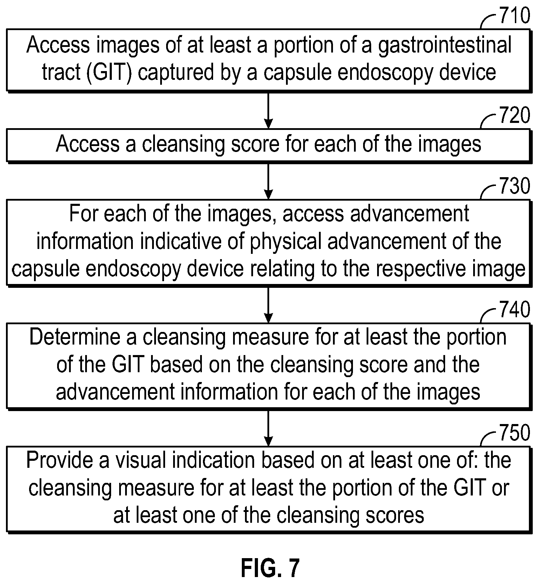

FIG. 7 is a flow diagram of an operation for determining a cleansing measure for a portion of a GIT or for the entire GIT. At block 710, the operation involves accessing images of at least a portion of a gastrointestinal tract (GIT) captured by a capsule endoscopy device. The images may be, for example, the stream of images 422-426n of FIG. 4. In various embodiments, the images of at least a portion of a GIT may be images of an entire GIT, images of a portion of a GIT, images of a colon, images of a small bowel, images of a portion of a colon, and/or images of a portion of a small bowel, among other things. At block 720, the operation involves accessing a cleansing score for each image of the images. The cleansing score may be, for example, an expected value cleansing score (e.g., 622, FIG. 6) or may be a cleansing score value in a continuous range (e.g., 624, FIG. 6). At block 730, the operation involves, for each of the images, accessing advancement information indicative of physical advancement of the capsule endoscopy device relating to the respective image. The advancement information may include motion information which indicates whether the CE imaging device which captured the image was static or in motion (e.g., 632, FIG. 6) and/or may include an estimate of how much the CE imaging device advanced between successive images to reach the location where the image was captured (e.g., 634, FIG. 6). For the first captured image, the advancement information may include an estimate of how much the CE imaging device advanced from a starting point to reach the location where the first image was captured.

At block 740, the operation involves determining a cleansing measure for a portion of the GIT or for the entire GIT based on the cleansing score and the advancement information for each of the images. Various embodiments are contemplated for the operation of block 740, and they will be described in the following paragraphs.

In various embodiments, the cleansing measure for a portion of a GIT or for the entire GIT may be an average of the cleansing scores for images which are not clean gastrointestinal wall images and/or whose movement information indicates that the CE imaging device was in motion when it captured the image. Accordingly, in various embodiments, images of clean gastrointestinal walls, and any cleansing scores for such images, are not used to determine the cleansing measure for the portion of the GIT or for the entire GIT. In various embodiments, images whose movement information indicates that the CE imaging device was static when it captured the image, and any cleansing scores for such images, are not used to determine the cleansing measure for the portion of the GIT or for the entire GIT. Static images may not be considered in determining the cleansing measure because they may capture the same information as adjacent or surrounding images, and the duplicated information may undesirably influence the cleansing measure. In various embodiments, rather than an average of cleansing scores, as mentioned above, the cleansing measure may be a median of cleansing scores or a minimum of cleansing scores, or another statistic.

In various embodiments, the cleansing measure for a portion of a GIT or for the entire GIT may be determined as:

$$\frac{\sum_{i=1}^{n}(score_i \cdot \Delta_i)}{\sum_{i=1}^{n}\Delta_i}$$

where n is a number of images whose cleansing scores will be used to determine the cleansing measure, $score_i$ is the cleansing score for image $i,$ and $\Delta_i$ is the advancement estimate for image $i$ which indicates how much the CE imaging device advanced between successive images to reach the location where the image was captured. For the first image of the n images, the advancement estimate $\Delta_1$ is an estimate of how much the CE imaging device advanced from a starting point to reach the location where the first image was captured. The term $\sum_{i=1}^{n}\Delta_i$ represents the total estimated advancement of the CE imaging device in capturing the n images. Thus, the term can be expressed as $\sum_{i=1}^{n}\Delta_i = \Delta_{total}$. Then, the cleansing measure can alternatively be expressed as:

$$\sum_{i=1}^{n}\left(score_i \cdot \frac{\Delta_i}{\Delta_{total}}\right).$$

Therefore, the cleansing measure may be expressed as a weighted sum of the cleansing scores for each image, where the weight for each image is the fraction of total advancement corresponding to the image. The cleansing measures described above are exemplary and variations are contemplated to be within the scope of the present disclosure.

With continuing reference to FIG. 7, at block 750, the operation involves providing a visual indication based on the cleansing measure for the portion of the GIT or for the entire GIT, and/or based on at least one of the cleansing scores for the images. In various embodiments, the visual indication may be a display of the cleansing measure or a display of at least one of the cleansing scores for the images. In various embodiments, the visual indication may be a color that represents the value of the cleansing measure, such as, for example, a red color for poor cleansing or a green color for excellent cleansing. In various embodiments, the visual indication may be a diagram of portions of a GIT in which colors are shown along the illustrated portions of the GIT to reflect the cleansing scores across the portions of the GIT. Such embodiments are exemplary, and variations are contemplated to be within the scope of the present disclosure.

The embodiment of FIG. 7 is exemplary, and variations are contemplated to be within the scope of the present disclosure. For example, in various embodiments, the operation may include a block between blocks 730 and 740 which excludes images which are clean gastrointestinal wall images and/or whose movement information indicates that the CE imaging device was static when it captured the image. Other types of images which are unhelpful to determining a cleansing measuring may also be excluded. Such and other variations are contemplated to be within the scope of the present disclosure.

The following describes various examples of the operation of FIG. 7 for determining a cleansing measure for various portions of a GIT or for the entire GIT. Referring also to FIG. 4, an example includes the illustration of FIG. 4 depicting the entire GIT 410, such that portion 412 represents pre-small bowel, portion 414 represents the small bowel, and portion 416 represents the colon. Each image in the stream of images 422-426n has associated cleansing score data and associated advancement information as described in connection with FIG. 6. Such associated data may be used by the operation of FIG. 7 to determine a cleansing measure for the pre-small bowel portion 412, a cleansing measure for the small bowel portion 414, and a cleansing measure for the colon 416. The cleansing measure for the pre-small bowel portion 412 would be based on the associated data for the images 422-422n. The cleansing measure for the colon portion 416 would be based on the associated data for the images 426-426n. The cleansing measure for the small bowel portion 414 be based on the associated data for images corresponding to that portion 414. In various embodiments, a cleansing measure can be determined for the entire GIT 410 based on the cleansing measures for each portion 412-416. For example, the cleansing measure for the entire GIT 410 may be a sum of the cleansing measures for each portion 412-416, or may be an average of such cleansing measures, among other things.

Another example includes the illustration of FIG. 4 depicting a GIT segment of interest 410, such as the small bowel. The small bowel may have any number of portions 412-416, such as five portions (e.g., duodenum, three tertiles, and terminal ilium) or one-hundred portions or one-thousand portions, among others. As persons skilled in the art will understand, the anatomy of the small bowel causes a CE imaging device to generally travel unidirectionally through the small bowel. Accordingly, existing techniques allow advancement estimates (e.g., 634, FIG. 6) to be generated for images of the small bowel. In accordance with aspects of the present disclosure, the operation of FIG. 7 may be applied to determine a cleansing measure for each portion of the small bowel. Specifically, the cleansing measure for the first portion 412 would be based on the associated data for the images 422-422n, the cleansing measure for the last portion 416 would be based on the associated data for the images 426-426n, and so on for the other portion(s) 414. In various embodiments, a cleansing measure can be determined for the entire small bowel 410 based on the cleansing measures for each portion. For example, the cleansing measure for the entire small bowel 410 may be a sum of the cleansing measures for each portion, or may be an average of such cleansing measures, among other things. The example described above is exemplary and the techniques can be applied to other GIT segments of interest other than the small bowel.

Another example includes the illustration of FIG. 4 depicting a GIT segment of interest 410, such as the colon. The colon generally has five main portions, as described above. As persons skilled in the art will understand, the anatomy of the colon allows a CE imaging device that is moving through it to rotate and roll, move backwards, rest in one place for a long period of time, and/or move very fast through various portions. through the small bowel. Due to these possible movements, advancement estimates (e.g., 634, FIG. 6) may not be available for images of the colon. In accordance with aspects of the present disclosure, the operation of FIG. 7 may be applied to determine a cleansing measure for each portion of the colon by excluding images which are clean gastrointestinal wall images and/or whose movement information indicates that the CE imaging device was static when it captured the image. The cleansing measure for the first portion 412 would be based on the associated data for the images 422-422n, the cleansing measure for the last portion 416 would be based on the associated data for the images 426-426n, and so on for the other portion(s) 414. In various embodiments, a cleansing measure can be determined for the entire colon 410 based on the cleansing measures for each portion. For example, the cleansing measure for the entire colon 410 may be a sum of the cleansing measures for each portion, or may be an average of such cleansing measures, among other things. The example described above is exemplary, and the techniques of the present disclosure may be applied to any number of portions of the colon or to other GIT segments of interest 410 other than the colon.

Accordingly, the description above describes using cleansing score data and advancement information to determine a cleaning measure for a portion of a GIT, for a GIT segment of interest, and/or for the entire GIT.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures.

The phrases "in an embodiment," "in embodiments," "in various embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

Any of the herein described operations, methods, programs, algorithms, or codes may be converted to, or expressed in, a programming language or computer program embodied on a computer or machine readable medium. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other metalanguages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

## Claims

1. A system for assessing gastrointestinal tract cleansing, comprising:

   one or more processors; and
   at least one memory storing instructions which, when executed by the one or more processors, cause the system to:

      access a plurality of images of at least a portion of a gastrointestinal tract (GIT) captured by a capsule endoscopy device;
      access a cleansing score for each image of the plurality of images;
      for each image of the plurality of images, access advancement information indicative of physical advancement of the capsule endoscopy device relating to the respective image;
      determine a cleansing measure for at least the portion of the GIT based on the cleansing score and the advancement information for each image of the plurality of images; and
      provide a visual indication based on at least one of: the cleansing measure for at least the portion of the GIT or at least one of the cleansing scores;

   wherein the cleansing measure for at least the portion of the GIT is computed as:

   $$\frac{\sum_{i=1}^{n}(score_i \cdot \Delta_i)}{\sum_{i=1}^{n} \Delta_i}$$

   wherein:

      $n$ is a number of images in the plurality of images,
      $score_i$ is the cleansing score for image $i$, and
      $\Delta_i$ is the advancement information for image $i$.

2. The system of claim 1, wherein the plurality of images includes a first image and successive images,

   wherein the advancement information for the first image of the plurality of images includes an estimate of how much the capsule endoscopy device has advanced from a beginning of the at least the portion of the GIT to reach a location where the first image was captured, and wherein for each of the successive images of the plurality of images, the advancement information includes an estimate of how much the capsule endoscopy device has advanced from a location where an immediate prior image was captured to reach a location where that image was captured.

3. The system of any one of the preceding claims, wherein the advancement information indicative of physical advancement of the capsule endoscopy device relating to an image is indicative of whether the capsule endoscopy device is static.

4. The system of claim 3, wherein in determining the cleansing measure for at least the portion of the GIT, the instructions, when executed by the one or more processors, cause the system to exclude cleansing scores for images whose advancement information indicates that the capsule endoscopy device is static.

5. The system of any one of the preceding claims, wherein the instructions, when executed by the one or more processors, further cause the system to: for each image of the plurality of images, access wall assessment information indicating whether the image is an image of only a clean wall of the at least the portion of the GIT, wherein in determining the cleansing measure for at least the portion of the GIT, the instructions, when executed by the one or more processors, cause the system to exclude cleansing scores for images whose wall assessment information indicates that the image is an image of only a clean wall.

6. The system of claim 4 or 5, wherein in determining the cleansing measure for at least the portion of the GIT, the instructions, when executed by the one or more processors, cause the system to compute the cleansing measure for at least the portion of the GIT as an average of the cleansing scores which were not excluded.

7. The system of any one of the preceding claims, wherein the cleansing score for each image of the plurality of images is based on a predetermined set of scores including:

a first score indicative of poor visualization of mucosa and indicative of at least one of: excessive content, excessive debris, excessive bubbles, excessive bile staining, excessive chyme staining, or excessive bleeding;
a second score indicative of moderate visualization of mucosa and indicative at least one of: moderate content, moderate debris, moderate bubbles, moderate bile staining, moderate chyme staining, or blurriness;
a third score indicative of good visualization of mucosa not through a big bubble and indicative at least one of: mild amount of debris, mild bubbles, mild bile staining, or mild chyme staining; and
a fourth score indicative of excellent visualization of mucosa not through a big bubble and indicative at least one of: minimal amount of debris, minimal bubbles, minimal bile staining, minimal chyme staining, no debris, no bubbles, no bile staining, or no chyme staining.

8. A computer-implemented method for assessing gastrointestinal tract cleansing, the method comprising:

accessing a plurality of images of at least a portion of a gastrointestinal tract (GIT) captured by a capsule endoscopy device;
accessing a cleansing score for each image of the plurality of images;
for each image of the plurality of images, accessing advancement information indicative of physical advancement of the capsule endoscopy device relating to the respective image;
determining a cleansing measure for at least the portion of the GIT based on the cleansing score and the advancement information for each image of the plurality of images; and
providing a visual indication based on at least one of: the cleansing measure for at least the portion of the GIT or at least one of the cleansing scores;
wherein the cleansing measure for at least the portion of the GIT is computed as:

$$\frac{\sum_{i=1}^{n}(score_i \cdot \Delta_i)}{\sum_{i=1}^{n}\Delta_i}$$

wherein:

$n$ is a number of images in the plurality of images,
$score_i$ is the cleansing score for image $i$, and
$\Delta_i$ is the advancement information for image $i$.

9. The computer-implemented method of claim 8, wherein the plurality of images includes a first image and successive images,

wherein the advancement information for the first image of the plurality of images includes an estimate of how much the capsule endoscopy device has advanced from a beginning of the at least the portion of the GIT to reach a location where the first image was captured, and wherein for each of the successive images of the plurality of images, the advancement information includes an estimate of how much the capsule endoscopy device has advanced from a location where an immediate prior image was captured to reach a location where that image was captured.

10. The computer-implemented method of claim 8 or 9, wherein the advancement information indicative of physical advancement of the capsule endoscopy device relating to an image is indicative of whether the capsule endoscopy device is static.

11. The computer-implemented method of claim 10, wherein determining the cleansing measure for at least the portion of the GIT comprises excluding cleansing scores for images whose advancement information indicates that the capsule endoscopy device is static.

12. The computer-implemented method of any one of claims 8 to 11, further comprising: for each image of the plurality of images, accessing wall assessment information indicating whether the image is an image of only a clean wall of the at least the portion of the GIT,
wherein determining the cleansing measure for at least the portion of the GIT comprises excluding cleansing scores for images whose wall assessment information indicates that the image is an image of only a clean wall.

**13.** The computer-implemented method of claim 11 or 12, wherein determining the cleansing measure for at least the portion of the GIT comprises computing the cleansing measure for at least the portion of the GIT as an average of the cleansing scores which were not excluded.

**Patentansprüche**

**1.** System zum Beurteilen der Reinigung des Magen-Darm-Trakts, umfassend:

einen oder mehrere Prozessoren; und mindestens einen Speicher, der Anweisungen speichert, die bei Ausführung durch den einen oder die mehreren Prozessoren das System zu Folgendem veranlassen:

Zugreifen auf eine Mehrzahl von Bildern mindestens eines Abschnitts eines Magen-Darm-Trakts, GIT, die durch eine Kapselendoskopie-Vorrichtung aufgenommen wurde;
Zugreifen auf eine Reinigungsbewertung für jedes Bild der Mehrzahl von Bildern;
für jedes Bild der Mehrzahl von Bildern Zugreifen auf Vorschubinformationen, die einen physischen Vorschub der Kapselendoskopie-Vorrichtung in Bezug auf das jeweilige Bild angeben;
Bestimmen eines Reinigungsmaßes für den mindestens einen Abschnitt des GIT basierend auf der Reinigungsbewertung und den Vorschubinformationen für jedes Bild der Mehrzahl von Bildern; und
Bereitstellen einer visuellen Anzeige basierend auf mindestens einem von Folgendem: dem Reinigungsmaß für den mindestens einen Abschnitt des GIT oder mindestens einer der Reinigungsbewertungen;
wobei das Reinigungsmaß für den mindestens einen Abschnitt des GIT berechnet wird als:

$$\frac{\sum_{i=1}^{n}(score_i \cdot \Delta_i)}{\sum_{i=1}^{n} \Delta_i}$$

wobei:

n eine Anzahl von Bildern in der Mehrzahl von Bildern ist,
$score_i$ die Reinigungsbewertung für Bild i ist, und
$\Delta_i$ die Vorschubinformationen für Bild i sind.

**2.** System nach Anspruch 1, wobei die Mehrzahl von Bildern ein erstes Bild und nachfolgende Bilder beinhaltet,

wobei die Vorschubinformationen für das erste Bild der Mehrzahl von Bildern eine Schätzung dessen beinhalten, wie weit die Kapselendoskopie-Vorrichtung von einem Anfang des mindestens einen Abschnitts des GIT vorgeschoben wurde, um eine Stelle zu erreichen, an der das erste Bild aufgenommen wurde, und
wobei für jedes der nachfolgenden Bilder der Mehrzahl von Bildern die Vorschubinformationen eine Schätzung dessen beinhalten, wie weit die Kapselendoskopie-Vorrichtung von einer Stelle, an der ein unmittelbar vorhergehendes Bild aufgenommen wurde, vorgeschoben wurde, um eine Stelle zu erreichen, an der dieses Bild aufgenommen wurde.

**3.** System nach einem der vorhergehenden Ansprüche, wobei die einen physischen Vorschub der Kapselendoskopie-Vorrichtung in Bezug auf ein Bild angebenden Vorschubinformationen angeben, ob die Kapselendoskopie-Vorrichtung statisch ist.

**4.** System nach Anspruch 3, wobei beim Bestimmen des Reinigungsmaßes für den mindestens einen Abschnitt des GIT die Anweisungen bei Ausführung durch den einen oder die mehreren Prozessoren das System veranlassen, Reinigungsbewertungen für Bilder auszuschließen, deren Vorschubinformationen angeben, dass die Kapselendoskopie-Vorrichtung statisch ist.

**5.** System nach einem der vorhergehenden Ansprüche, wobei die Anweisungen bei Ausführung durch den einen oder die mehreren Prozessoren ferner das System zu Folgendem veranlassen: für jedes Bild der Mehrzahl von Bildern Zugreifen auf Wandbeurteilungsinformationen, die angeben, ob das Bild ein Bild nur einer sauberen Wand des mindestens einen Abschnitts des GIT ist,
wobei beim Bestimmen des Reinigungsmaßes für den mindestens einen Abschnitt des GIT die Anweisungen bei Ausführung durch den einen oder die mehreren Prozessoren das System veranlassen, Reinigungsbewertungen für Bilder auszuschließen, deren Wandbeurteilungsinformationen angeben, dass das Bild ein Bild nur einer sauberen Wand ist.

**6.** System nach Anspruch 4 oder 5, wobei beim Bestimmen des Reinigungsmaßes für den mindestens einen Abschnitt des GIT die Anweisungen bei Ausführung durch den einen oder die mehreren Prozessoren das System veranlassen, das Reinigungsmaß für den mindestens einen Abschnitt des GIT als einen Durchschnitt der Reinigungsbewertungen zu berechnen, die nicht ausgeschlossen wurden.

7. System nach einem der vorhergehenden Ansprüche, wobei die Reinigungsbewertung für jedes Bild der Mehrzahl von Bildern auf einem vorbestimmten Satz von Bewertungen basiert, der Folgendes beinhaltet:

eine erste Bewertung, die auf eine schlechte Visualisierung der Schleimhaut hinweist und auf mindestens eines von Folgenden hinweist: übermäßigen Inhalt, übermäßige Ablagerungen, übermäßige Blasenbildung, übermäßige Gallenfärbung, übermäßige Chymusfärbung oder übermäßige Blutungen;

eine zweite Bewertung, die auf eine mäßige Visualisierung der Schleimhaut hinweist und auf mindestens eines von Folgenden hinweist: mäßigen Inhalt, mäßige Ablagerungen, mäßige Blasenbildung, mäßige Gallenfärbung, mäßige Chymusfärbung oder Unschärfe;

eine dritte Bewertung, die auf eine gute Visualisierung der Schleimhaut nicht durch eine große Blase hinweist und auf mindestens eines von Folgenden hinweist: geringe Menge an Ablagerungen, geringe Blasenbildung, geringe Gallenfärbung oder geringe Chymusfärbung; und

eine vierte Bewertung, die auf eine ausgezeichnete Visualisierung der Schleimhaut nicht durch eine große Blase hinweist und auf mindestens eines von Folgenden hinweist: minimale Menge an Ablagerungen, minimale Blasenbildung, minimale Gallenfärbung, minimale Chymusfärbung, keine Ablagerungen, keine Blasenbildung, keine Gallenfärbung oder keine Chymusfärbung.

8. Computerimplementiertes Verfahren zur Beurteilung der Reinigung des Magen-Darm-Trakts, wobei das Verfahren Folgendes umfasst:

Zugreifen auf eine Mehrzahl von Bildern mindestens eines Abschnitts eines Magen-Darm-Trakts, GIT, die durch eine Kapselendoskopie-Vorrichtung aufgenommen wurde;

Zugreifen auf eine Reinigungsbewertung für jedes Bild der Mehrzahl von Bildern;

für jedes Bild der Mehrzahl von Bildern Zugreifen auf Vorschubinformationen, die einen physischen Vorschub der Kapselendoskopie-Vorrichtung in Bezug auf das jeweilige Bild angeben;

Bestimmen eines Reinigungsmaßes für den mindestens einen Abschnitt des GIT basierend auf der Reinigungsbewertung und den Vorschubinformationen für jedes Bild der Mehrzahl von Bildern; und

Bereitstellen einer visuellen Anzeige basierend auf mindestens einem von Folgenden: dem Reinigungsmaß für den mindestens einen Abschnitt des GIT oder mindestens einer der Reinigungsbewertungen;

wobei das Reinigungsmaß für den mindestens einen Abschnitt des GIT berechnet wird als:

$$\frac{\Sigma_{i=1}^{n}(score_i \cdot \Delta_i)}{\Sigma_{i=1}^{n} \Delta_i}$$

wobei:

$n$ eine Anzahl von Bildern in der Mehrzahl von Bildern ist,

$score_i$ die Reinigungsbewertung für Bild i ist, und

$\Delta_i$ die Vorschubinformationen für Bild i sind.

9. Computerimplementiertes Verfahren nach Anspruch 8, wobei die Mehrzahl von Bildern ein erstes Bild und nachfolgende Bilder beinhaltet,

wobei die Vorschubinformationen für das erste Bild der Mehrzahl von Bildern eine Schätzung dessen beinhalten, wie weit die Kapselendoskopie-Vorrichtung von einem Anfang des mindestens einen Abschnitts des GIT vorgeschoben wurde, um eine Stelle zu erreichen, an der das erste Bild aufgenommen wurde, und

wobei für jedes der nachfolgenden Bilder der Mehrzahl von Bildern die Vorschubinformationen eine Schätzung dessen beinhalten, wie weit die Kapselendoskopie-Vorrichtung von einer Stelle, an der ein unmittelbar vorhergehendes Bild aufgenommen wurde, vorgeschoben wurde, um eine Stelle zu erreichen, an der dieses Bild aufgenommen wurde.

10. Computerimplementiertes Verfahren nach Anspruch 8 oder 9, wobei die einen physischen Vorschub der Kapselendoskopie-Vorrichtung in Bezug auf ein Bild angebenden Vorschubinformationen angeben, ob die Kapselendoskopie-Vorrichtung statisch ist.

11. Computerimplementiertes Verfahren nach Anspruch 10, wobei das Bestimmen des Reinigungsmaßes für den mindestens einen Abschnitt des GIT Ausschließen von Reinigungsbewertungen für Bilder umfasst, deren Vorschubinformationen angeben, dass die Kapselendoskopie-Vorrichtung statisch ist.

12. Computerimplementiertes Verfahren nach einem der Ansprüche 8 bis 11, ferner umfassend: für jedes Bild der Mehrzahl von Bildern Zugreifen auf Wandbeurteilungsinformationen, die angeben, ob das Bild ein Bild nur einer sauberen Wand des mindestens einen Abschnitts des GIT ist,

wobei das Bestimmen des Reinigungsmaßes für den mindestens einen Abschnitt des GIT Ausschließen von Reinigungsbewertungen für Bilder umfasst, deren Wandbeurteilungsinformationen angeben, dass das Bild ein Bild nur einer sauberen Wand ist.

13. Computerimplementiertes Verfahren nach Anspruch 11 oder 12, wobei das Bestimmen des Reinigungsmaßes für den mindestens einen Abschnitt des GIT Berechnen des Reinigungsmaßes für den mindestens einen Abschnitt des GIT als einen Durchschnitt der Reinigungsbewertungen umfasst, die nicht ausgeschlossen wurden.

**Revendications**

1. Système destiné à évaluer le nettoyage d'un tractus gastro-intestinal, comprenant :

   un ou plusieurs processeurs ; et
   au moins une mémoire stockant des instructions qui, lors de leur exécution par le ou les processeurs, amènent le système à :

   accéder à une pluralité d'images d'au moins une partie d'un tractus gastro-intestinal (GIT) capturées par un dispositif d'endoscopie par capsule ;
   accéder à un score de nettoyage pour chaque image de la pluralité d'images ;
   pour chaque image de la pluralité d'images, accéder à des informations de progression indiquant la progression physique du dispositif d'endoscopie par capsule par rapport à l'image respective ;
   déterminer une mesure de nettoyage au moins pour la partie du GIT sur la base du score de nettoyage et des informations de progression pour chaque image de la pluralité d'images ; et
   fournir une indication visuelle basée sur au moins un des éléments suivants : la mesure de nettoyage au moins pour la partie du GIT ou au moins un des scores de nettoyage ;
   dans lequel la mesure de nettoyage au moins pour la partie du GIT est calculée comme :

   $$\frac{\Sigma_{i=1}^{n}(score_i \cdot \Delta_i)}{\Sigma_{i=1}^{n}\Delta_i}$$

   où :

   n est un nombre d'images dans la plu-

ralité d'images,
score$_i$ est le score de nettoyage pour l'image i, et
$\Delta_i$ est l'information de progression pour l'image i.

2. Système de la revendication 1, dans lequel la pluralité d'images inclut une première image et des images successives,

   dans lequel les informations de progression pour la première image de la pluralité d'images comportent une estimation de la distance sur laquelle le dispositif d'endoscopie par capsule a progressé depuis un début au moins de la partie du GIT pour atteindre un emplacement où la première image a été capturée, et
   dans lequel, pour chacune des images successives de la pluralité d'images, les informations de progression comportent une estimation de la distance sur laquelle le dispositif d'endoscopie par capsule a progressé depuis un emplacement où une image immédiatement antérieure a été capturée pour atteindre un emplacement où cette image a été capturée.

3. Système de l'une quelconque des revendications précédentes, dans lequel les informations de progression indiquant la progression physique du dispositif d'endoscopie par capsule par rapport à une image indiquent si le dispositif d'endoscopie par capsule est statique.

4. Système de la revendication 3 dans lequel, lors de la détermination de la mesure de nettoyage au moins pour la partie du GIT, les instructions, lors de leur exécution par le ou les processeurs, amènent le système à exclure des scores de nettoyage pour des images dont les informations de progression indiquent que le dispositif d'endoscopie par capsule est statique.

5. Système de l'une quelconque des revendications précédentes, dans lequel les instructions, lors de leur exécution par le ou les processeurs, amènent en outre le système à : pour chaque image de la pluralité d'images, accéder à des informations d'évaluation de paroi indiquant si l'image est une image uniquement d'une paroi propre au moins de la partie du GIT,
   dans lequel, lors de la détermination de la mesure de nettoyage au moins pour la partie du GIT, les instructions, lors de leur exécution par le ou les processeurs, amènent le système à exclure des scores de nettoyage pour des images dont les informations d'évaluation de paroi indiquent que l'image est une image uniquement d'une paroi propre.

**6.** Système de la revendication 4 ou 5 dans lequel, lors de la détermination de la mesure de nettoyage au moins pour la partie du GIT, les instructions, lors de leur exécution par le ou les processeurs, amènent le système à calculer la mesure de nettoyage au moins pour la partie du GIT sous la forme d'une moyenne des scores de nettoyage qui n'ont pas été exclus.

**7.** Système de l'une quelconque des revendications précédentes, dans lequel le score de nettoyage pour chaque image de la pluralité d'images est basé sur un ensemble prédéterminé de scores incluant :

un premier score indicatif d'une mauvaise visualisation de la muqueuse et indicatif d'au moins un des éléments suivants : teneur excessive, débris excessifs, bulles excessives, coloration excessive de la bile, coloration excessive du chyme, ou saignement excessif ;
un deuxième score indicatif d'une visualisation moyenne de la muqueuse et indicatif d'au moins un des éléments suivants : teneur modérée, débris modérés, bulles modérées, coloration modérée de la bile, coloration modérée du chyme, ou flou ;
un troisième score indicatif d'une bonne visualisation de la muqueuse et non à travers une grosse bulle et indicatif d'au moins un des éléments suivants : légère quantité de débris, bulles atténuées, légère coloration de la bile, ou légère coloration du chyme ; et
un quatrième score indicatif d'une excellente visualisation de la muqueuse et non à travers une grosse bulle et indicatif d'au moins un des éléments suivants : quantité minimale de débris, bulles minimales, coloration minimale de la bile, coloration minimale du chyme, pas de débris, pas de bulles, pas de coloration de la bile, ou pas de coloration du chyme.

**8.** Procédé mis en œuvre par ordinateur pour évaluer le nettoyage d'un tractus gastro-intestinal, le procédé comprenant :

l'accès à une pluralité d'images d'au moins une partie d'un tractus gastro-intestinal (GIT) capturées par un dispositif d'endoscopie par capsule ;
l'accès à un score de nettoyage pour chaque image de la pluralité d'images ;
pour chaque image de la pluralité d'images, l'accès à des informations de progression indiquant la progression physique du dispositif d'endoscopie par capsule par rapport à l'image respective ;
la détermination d'une mesure de nettoyage au moins pour la partie du GIT sur la base du score de nettoyage et des informations de progression pour chaque image de la pluralité d'images ; et

la fourniture d'une indication visuelle basée sur au moins un des éléments suivants : la mesure de nettoyage au moins pour la partie du GIT ou au moins un des scores de nettoyage ;
dans lequel la mesure de nettoyage au moins pour la partie du GIT est calculée comme :

$$\frac{\Sigma_{i=1}^{n}(score_i \cdot \Delta_i)}{\Sigma_{i=1}^{n} \Delta_i}$$

où :

$n$ est un nombre d'images dans la pluralité d'images,
$score_i$ est le score de nettoyage pour l'image i, et
$\Delta_i$ est l'information de progression pour l'image i.

**9.** Procédé mis en œuvre par ordinateur de la revendication 8, dans lequel la pluralité d'images inclut une première image et des images successives,

dans lequel les informations de progression pour la première image de la pluralité d'images comportent une estimation de la distance sur laquelle le dispositif d'endoscopie par capsule a progressé depuis un début au moins de la partie du GIT pour atteindre un emplacement où la première image a été capturée, et
dans lequel, pour chacune des images successives de la pluralité d'images, les informations de progression comportent une estimation de la distance sur laquelle le dispositif d'endoscopie par capsule a progressé depuis un emplacement où une image immédiatement antérieure a été capturée pour atteindre un emplacement où cette image a été capturée.

**10.** Procédé mis en œuvre par ordinateur de la revendication 8 ou 9, dans lequel les informations de progression indiquant la progression physique du dispositif d'endoscopie par capsule par rapport à une image indiquent si le dispositif d'endoscopie par capsule est statique.

**11.** Procédé mis en œuvre par ordinateur de la revendication 10, dans lequel la détermination de la mesure de nettoyage au moins pour la partie du GIT comprend l'exclusion de scores de nettoyage pour des images dont les informations de progression indiquent que le dispositif d'endoscopie par capsule est statique.

**12.** Procédé mis en œuvre par ordinateur de l'une quelconque des revendications 8 à 11, comprenant en outre : pour chaque image de la pluralité d'images,

**EP 4 360 102 B1**

l'accès à des informations d'évaluation de paroi indiquant si l'image est une image uniquement d'une paroi propre au moins de la partie du GIT,

dans lequel la détermination de la mesure de nettoyage au moins pour la partie du GIT comprend l'exclusion de scores de nettoyage pour des images dont les informations d'évaluation de paroi indiquent que l'image est une image uniquement d'une paroi propre.

13. Procédé mis en œuvre par ordinateur de la revendication 11 ou 12, dans lequel la détermination de la mesure de nettoyage au moins pour la partie du GIT comprend le calcul de la mesure de nettoyage au moins pour la partie du GIT sous la forme d'une moyenne des scores de nettoyage qui n'ont pas été exclus.

FIG. 1

**FIG. 2**

**FIG. 3**

**FIG. 4**

EP 4 360 102 B1

**FIG. 5**

**610**

Image

**620**

**Cleansing Score Data**

**622**

Expected Value

**624**

Value in
Continuous Range

**626**

Wall Assessment
Information

**630**

**Advancement Information**

**632**

Motion Information

**634**

Advancement
Estimate

**FIG. 6**

```
                                                              ┌710
┌─────────────────────────────────────────────────────┐
│  Access images of at least a portion of a gastrointestinal │
│  tract (GIT) captured by a capsule endoscopy device   │
└─────────────────────────────────────────────────────┘
                              │
                              ▼                                ┌720
┌─────────────────────────────────────────────────────┐
│       Access a cleansing score for each of the images  │
└─────────────────────────────────────────────────────┘
                              │
                              ▼                                ┌730
┌─────────────────────────────────────────────────────┐
│         For each of the images, access advancement     │
│      information indicative of physical advancement of the │
│  capsule endoscopy device relating to the respective image │
└─────────────────────────────────────────────────────┘
                              │
                              ▼                                ┌740
┌─────────────────────────────────────────────────────┐
│   Determine a cleansing measure for at least the portion │
│      of the GIT based on the cleansing score and the   │
│      advancement information for each of the images    │
└─────────────────────────────────────────────────────┘
                              │
                              ▼                                ┌750
┌─────────────────────────────────────────────────────┐
│   Provide a visual indication based on at least one of: the │
│   cleansing measure for at least the portion of the GIT or │
│          at least one of the cleansing scores          │
└─────────────────────────────────────────────────────┘
```

FIG. 7

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020079696 A1
- WO 2020079696 A
- US 62867050
- US 63018890
- US 244988
- US 20190244351
- US 8792691 B